(19)

Europäisches
Patentamt
European
Patent Office
Office européen
des brevets

(11) **EP 2 051 177 A1**

(12) **EUROPEAN PATENT APPLICATION**

(43) Date of publication:
**22.04.2009 Bulletin 2009/17**

(51) Int Cl.:
***G06F 19/00*** (2006.01)

(21) Application number: **08020358.1**

(22) Date of filing: **11.02.2002**

(84) Designated Contracting States:
**AT BE CH CY DE DK ES FI FR GB GR IE IT LI LU MC NL PT SE TR**

(30) Priority: **09.02.2001 US 267970 P**
**16.02.2001 US 269220 P**
**20.09.2001 US 323600 P**
**20.09.2001 US 323599 P**

(62) Document number(s) of the earlier application(s) in accordance with Art. 76 EPC:
**02706238.9 / 1 360 483**

(71) Applicant: **The Trustees of Columbia University in the City of**
**New York**
**New York, NY 10027-6699 (US)**

(72) Inventors:
• **Rzhetsky, Andrey**
**New York, NY 10027 (US)**
• **Lo, Shaw-Hwa**
**New York, NY 10027 (US)**
• **Gomez Shawn, M.**
**New York, NY 10027 (US)**

(74) Representative: **Bachinger-Fuchs, Eva-Maria et al**
**Kopecky & Schwarz**
**Patentanwälte**
**Wipplingerstrasse 30**
**1010 Wien (AT)**

Remarks:
This application was filed on 22-11-2008 as a divisional application to the application mentioned under INID code 62.

(54) **Method for the prediction of molecular interaction networks**

(57) The present invention relates to a method for identifying unknown molecular interactions within biological networks based on representations of molecules as sets of conserved features. Such molecules include but are not limited to proteins and nucleic acid molecules which can be represented as collections of conserved features, such as domains and motifs in proteins. The method of the invention comprises computing the attraction probabilities between molecules followed by calculation of the probability of a biological network. The method of the invention can be applied across species, where interaction data from one, or several species, can be used to infer molecular interactions between molecules within or between organisms. The method of the present invention may be used to identify molecular interactions which can serve as drug screening targets.

EP 2 051 177 A1

**Description**

## 1. INTRODUCTION

The present invention relates to a method for identifying unknown molecular interactions within biological networks based on representations of molecules as sets of conserved features. Such molecules include but are not limited to proteins and nucleic acid molecules which can be represented as collections of conserved features, such as domains and motifs in proteins. The method of the invention comprises computing the attraction probabilities between molecules followed by calculation of the probability of a biological network. The method of the invention can be applied across species, where interaction data from one, or several species, can be used to infer molecular interactions between molecules within or between organisms The method of the present invention may be used to identify molecular interactions which can serve as drug screening targets.

## 2. BACKGROUND OF THE INVENTION

Recent achievements in genome sequencing, coupled with advances in cellular biology, have raised hopes for a greater understanding of the regulatory machinery of life. However, the transition from a linear, one-dimensional sequence of genes to an integrated, multi-dimensional model of metabolic and regulatory networks has yet to be made. Despite their importance, relatively little is understood about interactions between known genes and proteins, with a major complication being the general lack of data on the mechanism, rate, and even existence of genes and proteins. While progress has been made with advances in, for example, high-throughput two-hybrid studies and complementary interaction databases, a comprehensive view of these molecular interaction networks is still lacking. In fact, only recently have sufficient datasets become available to provide support for the analysis of such large-scale networks (Uetz et al., 2000, Nature 403:623-627; Xenarios et al., 2000, Nucleic Acid Res. 28:289-291).

Specialized databases for homology searches have recently been utilized in gene discovery projects and in recent years a number of efficient sequence comparison tools have been developed such as the BLAST (Basic Local Alignment Search Tool) family of programs designed for comparison of a single "search sequence" with a database (see Altschul et al., 1990, J. Mol. Biol. 215:403-410; Altschul et al., 1997, Nucleic Acids Res. 25:3389-3402), the family of Hidden Markov Model methods for comparison of a set of aligned sequences that usually represent a protein motif or domain with a database (e.g., Krogh et al., 1994, J. Mol. Biol. 235:1501-1531; Grundy et al., 1997, Biochem Biophys. Res. Commun. 231:760-6) and various other comparison tools (Wu et al., 1996, Comput. Appl. Biosci 12:109-118; Neuwald et al., 1995, Protein Sci. 4:1618-1632; Neuwald, 1997, Nucleic Acids Res. 25:1665-1677).

Other groups have aimed at capturing interactions among molecules through the use of programs designed to compare structures and functions of proteins (Kazic 1994, In: Molecular Modeling: From Virtual Tools to Real Problems, Kumosinski, T. and Liebman, M.N. (Eds.), American Chemical Society, Washington, D.C. pp. 486-494; Kazic, 1994, In: New Data Challenges in Our Information Age Glaesar, P.S. and Millward, M.T.L. (Eds.). Proceedings of the Thirteenth International CODATA Secretariat, Paris pp. C133-C140; Goto et al., 1997, Pac. Symp. Biocomput. p. 175-186; Bono et al., 1998, Genome Res. 8:203-210; Selkov et al., 1996, Nucleic Acids Res. 24:26-28).

## 3. SUMMARY OF THE INVENTION

The present invention relates to a method for identifying unknown molecular interactions within biological networks based on the representations of molecules as collections of features, where each feature is responsible for a specific interaction with another feature. For simplicity, the invention is described herein for protein interactions, however the method of the invention can also be used to identify additional types of molecular interactions.

In an embodiment of the invention, a method is provided for identifying unknown molecular interactions within biological networks based on the representation of proteins as collections of conserved domains and motifs, where each domain is responsible for a specific interaction with another domain. By characterizing the frequency with which specific domain-domain interactions occur within known protein interactions, the method of the invention permits the assignment of a probability to an arbitrary interaction between any two proteins with defined domains. Domain interaction data may be complemented with information on the topology of a biological network and is incorporated into the method by assigning greater probabilities to networks displaying more biologically realistic topologies. In an additional embodiment of the invention, Markov chain Monte Carlo techniques can be utilized for the prediction of posterior probabilities of intervention between a set of proteins, allowing its application to large data sets. The method of the invention can be applied across species, where interaction data from one, or several species, can be used to infer interactions between proteins. In addition, the method is can be analogously applied to other molecular data such as nucleic acid molecules including DNA and RNA molecules.

### 4. BRIEF DESCRIPTION OF THE DRAWINGS

**Figure 1.** Sampling of a network. The distribution of network topologies is modeled with a multinomial distribution. Individual bins contain a collection of networks with identical topologies. Networks within a given bin have probabilities defined on the basis of their edge composition.

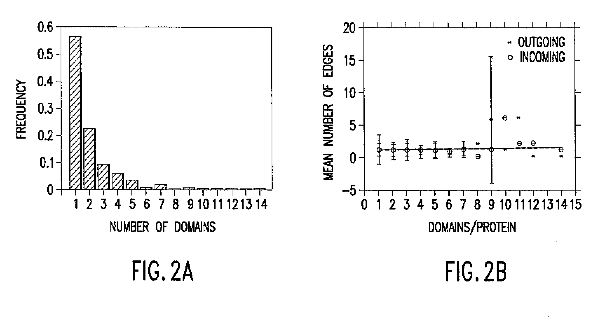

**Figure 2.** The number of domains per protein does not determine network connectivity. Data is from yeast network interaction data. (*a*) The frequency of proteins with a given number of domains. (*b*) The number edges outgoing ('x') or incoming ('o') to a protein is independent of the number of domains. Error bars represent 1 standard deviation. Regression lines are shown with slopes of 0.024 (intercept = 0.96) and 0.021 (intercept = 0.97) for outgoing and incoming edges respectively. The large deviation for the number of edges outgoing from proteins with 9 domains is due to the fact that only 3 data points comprise this set. All other points with 8 or more domains consist of a single sample (and thus have an undefined variance).

**Figure 3.** Probability distribution of vertices, with *k* incoming (open triangles) or outgoing (closed circles) edges. Edge distributions were calculated from the DIP database; they consisted of 1366 vertices with 1479 edges.

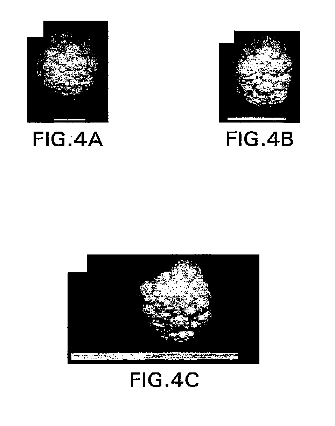

**Figure 4.** Scale-free (self-similarity) properties of a common cauliflower plant: it is virtually impossible to determine whether one is looking at a photograph of a complete vegetable or its part, unless an additional scale-dependent object (a match) is added. (*A*) Complete vegetable, (*B*) A small segment of the same vegetable, (*C*) Small part of the segment shown in ifigure (*B*). The same match was used in all three photographs for providing a sense of scale for otherwise scale-free structure. The idea originated from the book written by Peitgen and colleagues (Peitgen et al.,1992 in Chaos and Fractals: New Frontiers of Science, New York, Springer-Verlag). The photographs were obtained by direct scanning of the objects with *Compaq S$^4$* 100 scanner.

**Figure 5.** Log of network likelihood based only on network topology. *A, B,* Represent, respectively, a highly (overly) connected and minimally connected network. C, Depicts a more realistic (optimum) configuration. *D,* Shows a network with the same number of edges as (*C*) but with a less-favorable arrangement of incoming and outgoing edges. Networks with less-negative Log scores are more likely. Networks were created with Cutenet (Koike and Rzhetsky, 2000, Gene 259: 235-244).

**Figure 6.** Distribution of edges per vertex is scale invariant. The mean of'γ (~-2.3), along with 95% confidence intervals, is shown. See text for further details.

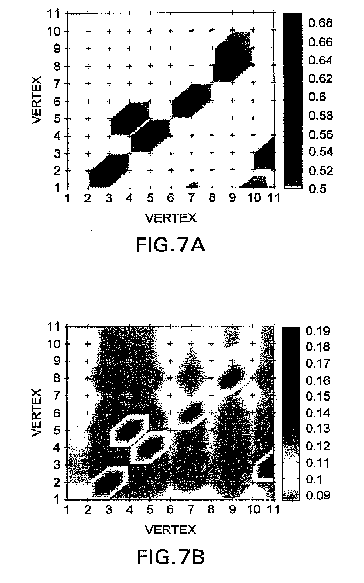

**Figure 7.** MCMC simulation of a small network. Vertices are 1-transcription factor BAS1 (gi|101447), 2-oxoglutarate dehydrogenase precursor (gi|1070439), 3-dihydrolipoamide S-succinyltransferase precursor (gi|2144399), 4-cell division control protein CDC43 (gi|2144611), 5-protein farnesyltransferase chain RAM2 (gi|266880), 6-pre-mRNA splicing factor PRP21 (gi|280467), 7-hypothetical protein YBL067C (gi|626480), 8-omnipotent suppressor protein SUP45 (gi|626763), 9-suppressor 2 protein (gi|72877), 10-transcription factor GRF10 (gi|82888), 11-dihydrolipoamide dehydrogenase precursor (gi|82983). **a,** Edge probabilities for the network based on domain-domain attraction probabilities alone. **b,** Posterior probabilities of all edges of the network after $10^9$ iterations of MCMC simulation. Red and blue colors represent probability above and below, respectively, the mean of all edge probabilities (in white). Note that only values at vertex intersections (+) have meaning-areas in between are interpolated and merely help to show gradients. Edges known to exist from the original data are ((3, 2), (4, 5), (5,4), (7,6), (9, 8), (9, 9), (10, 1), and (11,3)}. The percentage of rejected edges in MCMC computation was 82%.

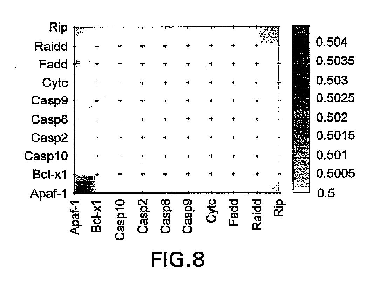

**Figure 8.** Prediction of interactions among 10 proteins that are involved in the human apoptosis pathway. Only probabilities based on domain-domain interactions alone are shown.

**Figure 9.** Known and predicted edges. Known edges are shown as open circles, while predicted edges are displayed as an "x".

**Figure 10.** Network posterior probabilities.

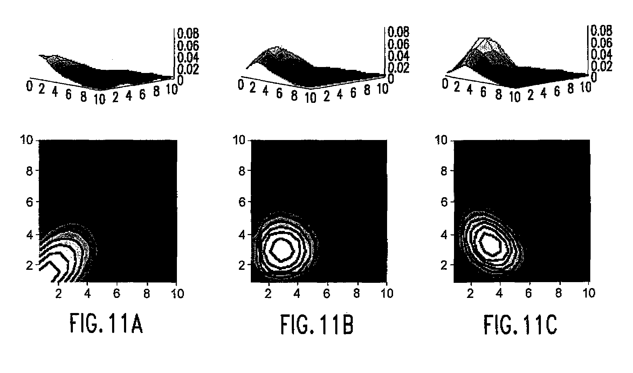

**Figure 11.** The negative multinomial distribution is an alternative to the multinomial. Parts (a) & (b) show the negative multinomial (surface plot above its corresponding contour plot) in comparison to the multinomial distribution in (c). For the multinomial, Pi = 0.25 and N = 14. For the neg. multinomial, Pi was set equal to 0.25 times a constant, with NP held constant. For part (a) the const = 4, while for part (b), const =$1 \times 10^{-6}$.

**Figure 12.** Probabilities of interactions between features. 12A. Tuple-Tuple. 12B Pfam domain-domain. 12C. Feature vector. 12D. Tuple-Pfam.

### 5. DETAILED DESCRIPTION OF THE INVENTION

Biological networks comprise proteins, nucleic acids, and small molecules as primary interacting elements. Functional areas that provide the ability for one molecule to interact with another are generally referred to as domains or motifs. For example, subsequences of DNA where specific proteins bind are one class of domain, as are the amino acid subsequence responsible for binding activity within the protein. Since genes are passive carriers of information, and because there are relatively few enzymatic or structural RNA molecules, the majority of important biological functions are carried out by proteins. Interactions between proteins are of particular interest, as they are responsible for the majority of "active" biological function. To date, protein-protein interactions are also the predominant type of interaction with significant quantities of supporting experimental data sets. Being linear sequences of amino acids at the level of primary structure, at the functional level, proteins can be broken down into segments that correspond to functional domains or conserved motifs. Like amino acids, these domains are discrete "letters," combinations of which give rise to the diversity of protein form and function.

For purposes of the present invention, the existence of a network connection between proteins, which may or may not involve a physical interaction between them, is a function of the domain composition of each. For convenience, non-protein network nodes are treated as single-domain proteins. Moving along a network pathway, a domain of an upstream protein may favor interaction with a domain of a downstream protein. In addition to a physical connection, the term "interaction" may also represent more general relationships between domains, e.g. information flow. The method of the present invention is based on the assumption that once a given pair of interactions has proven effective, nature will tend to re-use it in other networks within the same organism, as well as in other organisms. Thus, the method is based on quantifying, from data taken from known networks, the frequency with which a domain in one protein is observed immediately upstream or downstream of domains in another protein. This information is then used to infer the probability of unknown interactions.

## 5.1. DESCRIPTION OF THE METHOD

The present invention relates to a method for identifying unknown molecular interactions within biological networks based on the representation of proteins as collections of conserved domains and motifs, where each domain is responsible for a specific interaction with another domain. The method assigns probabilities to all possible networks found from a fixed number of vertices and provides each network with a probability value in such a way that networks having more features typical of real networks have higher probabilities.

The method of the present invention comprises representing a network as an oriented graph, $G = <V, E>$, where the vertices, $V$, correspond to proteins, and the edges $E$, correspond to interactions between proteins. Each vertex of the network is composed of one or more domains or motifs, which are identified through comparison with existing databases of protein domains (e.g. Pfam (Bateman et al., 2000, Nucleic Acids Res. 28:263-6). The frequency of separate occurrence of domains $d_m$ and $d_n$ in two connected vertices of a known network is used to infer probabilities of "attraction" $p(d_m, d_n)$, i.e., that an oriented edge will be found between these domains. As described in detail below, these probabilities are used to determine the probability of individual protein-protein interactions.

The method comprises two independent stochastic steps, and the probability of an individual network emerges as a product of the probabilities associated with these two steps. In the first step, every pair of proteins $i$ and $j$ may be connected to each other with an "attraction" probability $p_{ij}$, or not connected with probability $(1- p_{ij})$. One can imagine this process as being performed by a machine that, for every pair of vertices, tosses imaginary biased coins, each coin specific to a particular pair of proteins. If it is heads, an edge between the two vertices is formed; if it is tails, it does not. The coin is biased by prior information about the domains in each of the vertices, leading to some edges having a probability greater than 0.5 (attraction) and some edges less than 0.5 (repulsion). For a network with $|V|$ vertices, there are $2^{|V||V|}$ possible networks with oriented edges. The probability of a single network with the particular edge set E is defined as:

$$P(E) \;=\; \prod_{e_{ij} \in E} p_{ij} \prod_{e_{kl} \notin E} [1 - p_{kl}]. \qquad (1)$$

Using this process, one can assign a probability to any configuration of edges between a set of vertices. Networks containing many high-probability edges will have higher probabilities $P(E)$.

In the second step, networks are sorted into a finite number of bins, each corresponding to a particular "network topology." In this case, "network topology" is defined as the particular distribution of edges coming into and out of each vertex of the network. For a given number of vertices, it is possible to have a large number of edge configurations that are characterized by the same topology, so each bin represents a collection of networks with identical topologies. The number of incoming edges, or *indegree*, of a vertex in an oriented graph is the number of oriented edges that end at this vertex. Similarly, the *outdegree* of a vertex is the number of oriented edges that start at this vertex. For a pair of proteins connected with a single oriented edge, the upstream protein has a single outgoing edge while the downstream protein possess a single incoming edge. For each network the number of vertices that have outdegree zero, $n_0^{out}$, one,

$n_1{}^{out}$, two, $n_2{}^{out}$, and so on to $n_N{}^{out}$ (where the subscript indicates the number of edges, and N is the total number of vertices in the graph) is computed. Similarly, the numbers of vertices with indegrees 0, 1,2,... .N can be computed. Into one bin all networks with identical sets $\{n_x{}^{in}\}$ and $\{n_y{}^{out}\}$ are added. For each bin a sampling probability, $P(\{n_x{}^{in}\}, \{n_y{}^{out}\})$ is defined that is computed as the product

$$P(\{n_x^{in}\}; \{\pi_x^{in}\}, |V|) \times P(\{n_y^{out}\}; \{\pi_y^{out}\}, |V|), \qquad (2)$$

where

$$P(\{n_z\}; \{\pi_z\}, |V|) = \frac{|V|!}{n_0! \ldots n_N!} \prod_{z=0}^{N} \pi_z^{n_z}. \qquad (3)$$

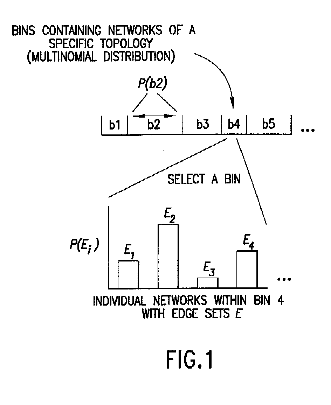

The probability distributions $\pi_x{}^{in}$ and $\pi_y{}^{out}$ give the probability of a network having x incoming and $y$ outgoing edges respectively. These distributions are explained in detail below. Finally, the second step is finished with a random (multinomial) sampling of a bin with probability $P(\{n_x{}^{in}\}, \{n_y{}^{out}\})$, and then random (uniform) sampling of a network from within that bin (see Figure 1). At the topological level, it is not possible to distinguish between the situations where (a) protein 1 has $n_i$ inputs and protein 2 has $n_j$ inputs and (b) protein 1 has $n_j$ inputs and protein 2 has $n_i$ inputs. Rather, the distinction is made at the level of individual edges. Each individual edge has a probability associated with it, and thus so does the complete set of edges that make up a given network $P(E)$. A network topology is automatically defined when given this same set of edges $E$. The probability of this particular topology, however, is determined separately and may or may not be biologically realistic. It is not necessary to distinguish between (a) and (b) above because *topologically* they are identical. They are not identical, however, at the level of individual edges. Each of these edges in (a) and (b) will have a different probability associated with them, with presumably one version of (a) or (b) being the correct, and thus most favorable one.

To verify that the product of the former two stochastic steps would give the probability of sampling any given network the following equation may be utilized:

$$P(E) \times P(\{n_x^{in}\}; \{\pi_x^{in}\}, |V|) \times P(\{n_y^{out}\}; \{\pi_y^{out}\}, |V|). \quad (4)$$

Networks with both favorable sets of edges E and favorable topologies will have the highest probabilities.

It is important to note that real biological networks have a very characteristic topology that distinguishes them from the vast majority of arbitrary random networks. Therefore, in a situation where information about protein domain interactions is far from being complete, a restriction on acceptable network topology is used to improve the prediction ability of the algorithm described therein.

Proteins are viewed as "collections of domains" where each individual pair of domains, $d_m$ and $d_n$, has a probability of getting attracted, $p(d_m, d_n)$. If $p(d_m, d_n) > 0.5$, the domains "attract" each other, while for $p(d_m, d_n) < 0.5$, the domains "repel" each other. Considering a pair of multidomain proteins $i$ and $j$, where $v_i$ and $v_j$ are the sets of domains for each protein (a domain of each type occurs in $v_i$ no more than once, even if the ith protein has multiple domains of the same kind), it is assumed that the probability of attraction (= edge probability) between these proteins is given in terms of domain attraction probabilities as

$$p_{ij} = \sum_{d_m \in v_i} \sum_{d_n \in v_j} \frac{p(d_m, d_n)}{|v_i||v_j|}. \qquad (5)$$

Interactions between proteins published in the research literature have strikingly different reliabilities. This is in part due to the fact that it is uncommon to publish the negative results of an experiment. As a result, the presence of an interaction between proteins is usually backed by multiple experiments while the absence of interaction may correspond to a failed experiment or just the absence of experiments at all (the only exclusion from this observation is exhaustive two-hybrid screening where all results, both positive and negative, are reported). Therefore, the probabilities of "attraction" between two domains should be estimated in such a way that the absence of a connection is treated as the absence of data, while the counts of known connections are used to estimate the probabilities. That is, for domains $d_m$ and $d_n$, attraction probability is computed as

$$p(d_m,\ d_n) = \frac{1}{2}\left(1 + \frac{k_{mn}}{k_m k_n + \Psi}\right), \tag{6}$$

where $\Psi$ is a positive real-valued pseudocount, $k_{mn}$ is the number of edges in the training set that contain at least one domain $d_m$ at the vertex of edge origin and at least one domain dn at the vertex of edge destination, $k_m$ is the number of distinct vertices that contain at least one domain $d_m$, and $k_n$ is the number of distinct vertices that contain at least one domain $d_n$. For this work $\Psi = 1$ is chosen, which can be increased (or decreased) if one wants to require the accumulation of greater amounts of data before the prior becomes significantly altered. As an example, if there are two upstream proteins with domain *m* and two downstream proteins each with domain n, a perfect correspondence between protein domains and the existence of an edge would lead to $k_{mn} = 4$ (all possible edges exist), $k_m = 2$, $k_n = 2$ and $p(d_m, d_n) =$ 0.9 (assuming that $\Psi = 1$).

As a result, this formulation assigns probabilities greater than 50% to edges that have known connections, and probabilities *equal* to 50% to edges that have no known connections. In the absence of experimental observations $(k_m = k_n = k_{mn} = 0)$, the probability of an edge forming between any two domains is exactly 50%, which in turn leads to a probability of 50% for forming an edge between any two proteins, regardless of the number of domains in each of them. In the absence of data, *all networks* can be assigned a non-zero probability. While the model allows for it, the current methodology (specifically, Equation 6) does not generate probabilities of less than 0.5 for domain-domain interactions. While the probability range could be expanded from 0 - 1, the compressed scale of 0.5 - 1 does not affect the results. Additionally, the method may be modified, combining the collection of appropriate data (e.g. negative experimental results, appropriate 2-hybrid data, etc.), with the range of 0 - 0.5 for modeling "repulsive" effects between domains.

The method of the present invention assigns a probability to every possible network with |V| vertices. This probability is based on both local and global network properties. At the local level, the probability of a vertex having an interaction with another vertex is dependent on the domain composition of each. If, as previously determined by training data, the set of domains in one protein are likely to be attracted to those of another protein, the probability of an edge existing between the two vertices increases to a value greater than 0.5. If no information is available about the likelihood of interaction for the set of domains contained in both upstream and downstream vertices, the probability of an edge forming between the two is taken to be 0.5. The probability of the network (based solely on local properties) is modified based on how well it represents real biological networks. For example, networks with topologies (distribution of incoming and outgoing edges per vertex) that are more biologically realistic are given higher likelihoods. The probability of any given network is the product of both the local and global probabilities.

### 5.2. ESTIMATING THE PARAMETERS RELEVANT TO THE TOPOLOGY OF REAL NETWORKS

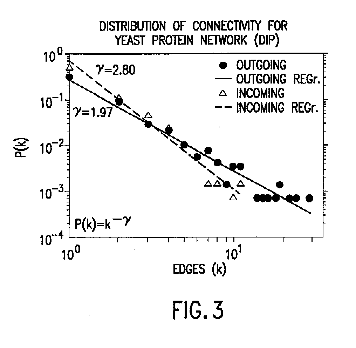

When the parameters $\{\pi_x^{in}\}$ and $\{\pi_y^{out}\}$ from the DIP dataset were estimated, it was found that the estimated values for both sets followed a power-law distribution (Figure 3). This means that, in logarithmic coordinates, the relationship between the number of connections per vertex and the proportion of vertices with that many connections is *linear* for both incoming and outgoing edges. This power-law distribution is a property of scale-free systems. These systems have properties or behaviors that are invariant across changes in scale. A demonstration of this phenomenon is shown in Figure 4, where it is not possible to determine the scale of the object (in this case, cauliflower) without a reference object. This property has also been seen in networks (Albert et al. 2000 Nature 406:378-382; Barabasi and Albert, 1999 Science 286:509-512), including social and non-biological networks, where the probability of a vertex having *k* incoming or outgoing edges is given by

$$\pi_k = ck^{-\gamma}, \tag{7}$$

but with the values of $\gamma$ and $c$ being different for incoming and outgoing edges. For the outgoing edges of a network, a linear fit at logarithmic scale gives estimates of c = 0.30 and $\gamma = 1.97$, whereas incoming edges are distributed with c =

0.56 and $\gamma$ = 2.80. For this work, the power law for $\pi_k$ only for non-zero $k$ was used; the values of $\pi_0^{in}$ and $\pi_0^{out}$ were estimated as

$$\pi_0^{in} = 1 - \sum_{k=1}^{\infty} \pi_k^{in}, \quad \pi_0^{out} = 1 - \sum_{k=1}^{\infty} \pi_k^{out}. \qquad (8)$$

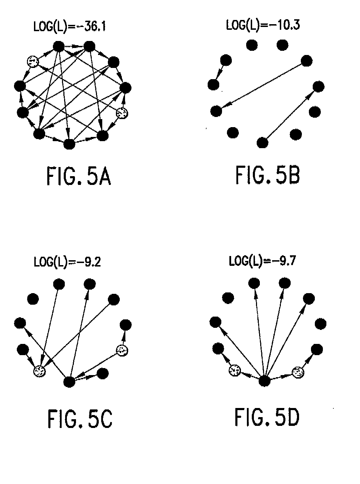

Examples of the influence of network topology on the likelihood of a given network are shown in Figure 5.

6. EXAMPLE: <u>PREDICTION OF PROTEIN-PROTEIN INTERACTIONS</u>

In order to test the efficacy of the model described herein, networks with large numbers of known protein-protein interactions were utilized. For this work, *Sacchromyces cerviseae* protein-protein interactions taken from the Database of Interacting Proteins (DIP; http://dip.doe-mbi.ucla.edu/) (Xenarios et al., 2000 Nucleic Acids Research 28: 289-291) were used. The domains involved in each interaction were determined by analyzing protein sequences with hmmpfam (Bateman et al., 2000 Nucleic Acids Research 28:263-6), a publicly available software tool that referenced 2015 domains at the time of this analysis. A total of 638 protein-protein interactions (all with at least 1 domain) were analyzed, and then used to determine the domain-domain interaction probabilities. Data (in this case a list of undirected protein-protein interactions) used for studying the effect of vertex removal on network edge distributions were taken from the Fields Lab Home Page (http://depts.washington.edu/sfields/).

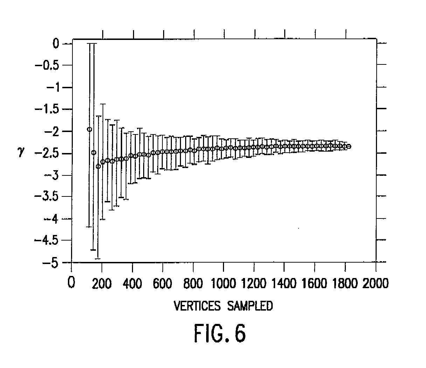

**The yeast protein network is scale-free.** It is known that the observed power-law behavior for the distribution of edge types within the network implies a scale-free system. To provide another means of verification, the value $\gamma$ for a large network (1823 vertices) was determined. A bootstrap procedure was then ran where for 200 iterations, where 30 vertices were randomly removed from the network and the value of $\gamma$ and 95% confidence intervals were determined for each. After this was completed, 60 vertices were removed and the process repeated. This was repeated until the final 200 iterations with 113 total vertices in the network. The effect of vertex removal on $\gamma$ is shown in Figure 6 (mean of $\gamma$ and 95% confidence intervals displayed), and shows that this network is remarkably scale-invariant. This implies that knowledge of the topology of a small part of a network should provide a reliable means of estimating the complete network's topology.

**Cross Validation**. Cross-validation was used to determine the effectiveness of the model in predicting the overall network configuration. Cross-validation is a general technique for evaluating the efficiency (and hence, validity) of statistical algorithms. It typically involves dividing a data set into two disjoint subsets, one of which is used for training, with the other being used for model validation. The jackknife version of cross-validation was used, where the training set consists of the complete network minus a single, specified edge. The likelihood of the complete graph (the model validation set of data) was compared to that of the complete graph minus one edge. If the likelihood of the full network was greater than the likelihood of the reduced network, the edge was considered to be positively predicted. This step was performed iteratively until all edges had been considered. Analysis of the test network indicated that the model predicted 93% of the known 642 edges used in the test; the remaining 7% *represent false negatives.* The rate *of false positives* was similarly estimated as ~10% by starting with the full known network and attempting to add a single edge between unconnected vertices. Note that this measure of false positives assumes that all edges not included in the true network should not exist. Currently, it cannot determined which, if any, of the false-positive edges correspond to true - but currently unknown - connections. While it should be possible to achieve even greater accuracy by including more data in the training set, these results demonstrate that the model is valid and is capable of making reasonably accurate predictions.

**Markov chain Monte Carlo.** For nearly all species, many interactions within a biological pathway are currently unknown. Since the method of the present invention permits computation of the probability for any possible arrangement of edges that connect a set of vertices, a Markov chain Monte Carlo (MCMC) simulation approach (Gilks et al, (editors) in Markov chain Monte Carlo in Practice. Chapman & Hall, New York; Hastings ,1970 Biometrika 57:97-109) can be implemented; which allows computation of posterior probabilities for all edges while effectively sampling from the astronomically large number of possible networks.

A reversible-jump methodology (Green, 1995 Biometrika 57 82:711-732) typical for Bayesian model selection was implemented, treating different networks as alternative statistical models. A uniform prior distribution was chosen over all networks, because, without additional information, there is no reason to prefer one network over another. Starting with an arbitrary network, the algorithm either adds or removes, with equal probability, a defined number of edges. Edges to be added or deleted are respectively sampled from the pool of edges that are included or excluded from the current network, with the probability of selecting any given edge dependent on only the number of edges from which to choose.

Adding or removing edges in this manner, the system jumps from network X to a new network Y. The proposed new state Y is sampled from the proposal distribution, q(b|a). The new network Y is then accepted with probability

$$\alpha(X,\ Y) = \min\left\{1, \frac{L(Y)q(X\mid Y)}{L(X)q(Y\mid X)}\right\}, \qquad (9)$$

where L(.) is the likelihood of the given network. If the proposed new state is accepted, then network *Y* becomes the current network; otherwise, the old network *X* remains as the current model. The stochastic process moves through the space of possible networks, on average keeping each edge in an on or off state in proportion to the posterior probabilities of this edge being present or absent in the correct network.

As a small-scale example, a group of 11 yeast proteins known to interact with at least one other member of the group was selected, and an attempt was made to predict edges (Figure 6). The probabilities of a given edge, based on domain-domain interactions alone, are shown in part a. Note that all edges except (7,1) (x-axis, *y*-axis) are found in the original data. The posterior probability estimated through simulation is shown in part b, and all known edges except (10, 1) are predicted reliably. This result is not merely a sampled version of 6a; rather, it incorporates the constraints imposed by the edge distributions on the topology of the network. Thus edges (7, 1) and (10, 1) are not supported with high confidence due to their low domain-domain interaction probabilities and to the influence of the edge distributions. The effect of topology constraints can also be seen where regions of low probability (e.g. the vicinity of (4, 8)) are associated with proteins that already have a high-probability edge; addition of a second edges is unlikely. The nonsymmetrical pattern is due to differences between the outgoing and incoming edge distributions. Although they are easily differentiated from unlikely edges, all likely edges have relatively low posterior probabilities.

For very small systems, a significant amount of information can be gained simply from looking at the edge probabilities between a given set of vertices, with very little additional information coming from topology information. However, use of the MCMC method described here should be particularly valuable for the prediction of large networks, where large amounts of protein interaction data with complicated domain architectures (such as those of higher organisms), and a computationally intensive number of network topologies, is the norm.

As a further example of the application of domain -domain interaction information, 10 proteins known to function in the human apoptosis pathway were selected from the KEGG database (Goto et al., 1997 Pac Symp Biocomput 175-86). As is obvious from Figure 7, few edges were supported by yeast training data; however, the most strongly predicted interaction was of Apaf-1 interacting with itself. A search of the signal-transduction literature revealed that Apaf-1 does, in fact, self-associate (Benedict et al., 2000 J. Biochem Chem 275:8461-8; Hu et al., 1998 J. Biol Chem 273: 33489-94). To date this association is not known, and was not described within KEGG. While not being able to predict the known network, this example is remarkable given the small amount of domain-domain interaction data available for training; and demonstrates the potential application of this method to predicting interactions across species. Accumulation of interaction from more complicated organisms should greatly enhance these predictions.

Based on the simple concepts of domain composition and network topology, the present invention permits both characterization and prediction of both known and unknown protein interactions within a given species, and potentially, across species. Markov chain Monte Carlo techniques described earlier provide a computationally feasible way to calculate the posterior probability of a network given data as:

$$P(\text{network}_i\mid \text{data}) = \frac{P(\text{data}\mid \text{network}_i)P(\text{network}_i)}{\sum_{\text{all networks}}P(\text{data}\mid \text{network}_j)P(\text{network}_j)}. \qquad (10)$$

In addition, techniques utilizing genetic algorithms, maximizing likelihood, and simulated annealing can be used to calculate the posterior probability of a network. Such techniques are know to those of skill in the art. While a uniform prior distribution over all possible networks is assumed, the method does not require this. Furthermore, additional information (in the form of priors) can be added into the calculation as it becomes available.

In the study of regulatory pathways, the method of the present invention could significantly reduce the number of required experiments by identifying a few most likely hypotheses. Such experimental analysis is itself an empirical way of validating the model, and can facilitate likewise design experiments for validation. Improvements could include additional interaction data and the introduction of more domains for assignment to protein segments. The method may be further enhanced by allowing the introduction of repulsion effects, which are implemented by allowing probabilities of less than 0.5 for domain -domain interactions. This information can be gathered from experiments (past and future) as well as from experts in the field. Also, the creation of pseudo-domains for characterizing non-protein substances and small molecules would allow their analysis within the network.

Despite the lack of data on various molecular parameters (e.g. rate constants), modeling at this level of detail may provide significant benefits. For example, von Dassow and colleagues (Von Dassow et al., 2000 Nature 406:188-92) have recently described a nonlinear differential-equation model for the simulation of the segment polarity network within *Drosophila.* Surprisingly, they found that the performance of this network was not dependent on the value of specific kinetic parameters, but rather achieved stability through the topology of the network itself.

Of special interest is the finding that the connectivity of vertices appears to follow a power-law distribution, exhibiting scale-free behavior. Such behavior implies that the points where a newly added protein is connected to the network will occur preferentially with proteins having greater numbers of preestablished connections (*i.e.*, a "rich get richer" phenomenon). This phenomenon has been observed within metabolic networks, and most recently, studies by Jeong and colleagues have also demonstrated the scale-free nature of the protein-protein interaction network within yeast described here (Jeong et al., Nature 411:41-42; Jeong et al., 2000 Nature 407:651-4). The presence of a large number of connections may indicate a fundamentally more important, or more versatile, protein function; a possible real-world example being the protein p53.

## 7. EXAMPLE: <u>PREDICTION OF HUMAN PROTEIN-PROTEIN INTERACTIONS</u>

A combined dataset of protein-protein interaction data for both *Sacchromyces cerviseae* and *Homo sapiens* was used. The Pfam database (Pfam 6.2; 2773 domains) and the HMMER package were used to determine the domains within each proteins (0.01 significance threshold). For the yeast data, a comprehensive list of interactions downloaded from Stanley Field's lab home page (http://depts.washington.edu/sfields/) was utilized. This data included interactions from a number of sources (Xenarios et al., 2001 Nucleic Acids Research 28:289-91; Ito et al., 2000 Proc. Natl. Acad Sci. USA 97:1143-7; Uetz et al., 2000 Nature 403:623-7). A total of 708 protein-protein interactions were analyzed from yeast, all of which had at least 1 domain. For human data, a set of 778 interactions downloaded from the Myriad Genetics Pronet Online web site (http://www.myriad-pronet.com/) was used. For the analysis, an attempt was made to predict interactions in a set of 40 human proteins known to form a connected network, and which had not been included in the original training data set.

An attempt was made to predict interactions between a set of 40 human proteins known to form a fully connected network, with some of the proteins involved in the process of apoptosis. Except for the requirement that all proteins of the network must be defined by at least one domain, this network was chosen at random. Proteins used in this analysis (and their indices in all figures) are: 1) ANT2, 2) APP (695), 3) B-CAT, 4) BAG3, 5) BAK, 6) Bax-beta, 7) Bcl-xL, 8) BCL2A1, 9) Bc12-alpha, 10) Calsenilen, 11) CAV1, 12) CHIP, 13) CIB, 14) D-CAT, 15) DRAL, 16) FLN1, 17) FLNB, 18) GAPCenA, 19) GDI1, 20) GDI2, 21) GGTB, 22) GTPBP1, 23) HSPA4, 24) HSPA8, 25) KSR1, 26) MCL1, 27) MRJ, 28) PSAP, 29) PKP4, 30) PLCG1, 31) PS1 (467), 32) PS2 (448), 33) QM, 34) RAB11A, 35) RAB3A, 36) RAB5A, 37) RAB6, 38) RAB6KIFL, 39) TF, 40) TTC1.

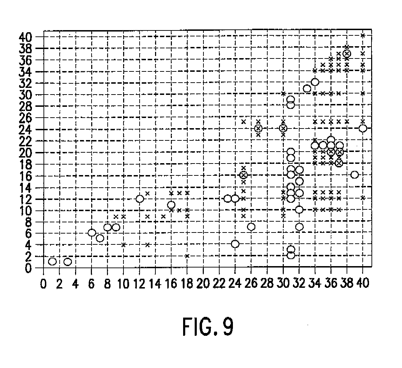

Edge probabilities based on domain-domain interaction data alone indicated that 97 edges had probabilities > 0.5 (see Figure 9). It was assumed that edges were not directed and thus the matrix shown here is symmetric. A total of 44 edges were in the original data set. Of these 44 edges, 8 are observed in the predicted 97 with probabilities > 0.5. Three out of eight interactions were involved in the heat shock pathway (read as (Y-axis, X-axis) on the figure); CHIP (12, 12) self-interaction, HSPA8-MRJ (24, 27), and HSPA8-PLCG1 (24, 30). The remaining 5 included FLN1-KSR1 (16,25), PS2-CIB (32, 13), GDI2-RAB6 (20, 37), RAB6-GAPCenA (37, 18), and RAB6-RAB6KIFL (37, 38).

To see if any of the remaining 89 predicted edges represent known edges, a brief literature search was attempted. While often requiring significant expertise in a given pathway to adequately evaluate these results, it was still possible to find obvious successes. The predictions of GDI1 (Guanine Nucleotide Dissociation Inhibitor, vertex 19) interacting with Rab11A, Rab3A, Rab5A, and Rab6 (vertices 34, 35, 36, 37 respectively) are in fact correct, and again not in the original data (Hutt et al., 2000 J. Biol Chem 275:18511-9; Wu et al., 1998 J. Biol. Chem 273:26931-26938; Ullrich et al., 1993 J. Biol. Chem 268:18143-50).

The prediction of CHIP interacting with TTCI (tetratricopeptide repeat domain 1) (12, 40) is also understandable (though likely not a correct prediction, it may also be questionable in the original data) as the tetratricopeptide domain is a common protein-protein interaction motif, and a number of TPR containing proteins are known to interact with members of the heat shock protein family (Ballinger et al., 1999 Mol Cell Biol 19:4535-45). While purely speculative, the interaction of CIB (calcium and integrin binding protein with FLN1 (filamin) is interesting, as filamin has recently been shown to be a scaffold protein that interacts with calcium receptor and other cell signaling proteins (Awata et al., 2001 J. Biol. Chem 4:4).

A Markov Chain Monte Carlo (MCMC) simulation approach for computing the posterior probabilities of all edges within the network (Gilks et al., 1996 Markov chain Monte Carlo in Practice New York: Chapman & Hall/CRC; Hastings, 1970 Biometrika 57: 97-109) was used (for program see Appendix A). This approach, particularly useful in generating posteriors from complicated distributions, allowed adequate sampling from the astronomically large number of possible network configurations (for $|V|$ vertices there are $2^{|V||V|}$ possible networks). In this approach a uniform prior distribution over all networks was used, as no prior information was known that would cause one to prefer one network over another. Starting with an arbitrary network, and using a reversible-jump methodology (Green, 1995 Biometrika 82: 711-732) edges were both added and removed at each iteration of the algorithm. Addition and removal of edges moves the network from the current state Xto a proposed state Y. Using a symmetric proposal distribution, the new state is accepted with probability

$$\alpha(X,\ Y) = \min\left\{1, \frac{L(Y)q(X\mid Y)}{L(X)q(Y\mid X)}\right\}, \qquad\qquad (11)$$

where $L(.)$ is the likelihood of the network. If the proposed state is accepted, it becomes the current state. This method thus samples networks from the space of all possible networks while keeping each edge occupied, or unoccupied over time, in proportion to its posterior probability.

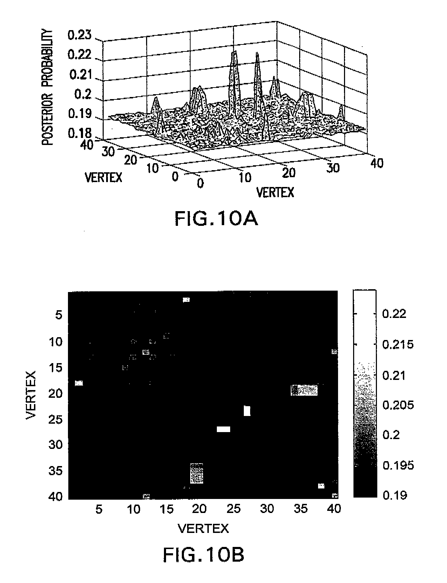

The posterior distribution generated from approximately $10^7$ samples is shown in Figure 10A-B. In 10A it can be seen that a few edges are readily apparent; rising well above the surrounding background. The two tallest peaks are of the HSPA8-MRJ interaction. Edges such as these show up rapidly in the simulations, while low-probability edges can take considerably greater amounts of sampling to distinguish them from background. Figure 10B shows the posterior probabilities for each edge of the network. The lower probability (darker) "lines" running horizontally at vertices 20 and 27 and vertically along vertex 27 show the influence of the nonsymmetrical edge distributions. For example, since vertex 27 has a high probability connection, the edge distribution tends to suppress the addition of new edges to the same vertex. Of course any vertex can have multiple incoming and outgoing edges, however due to the scale-free property of these networks, highly connected vertices are relatively rare.

As discussed in the method description, multinomial distribution is currently used to characterize the distribution of edges going into and out of each vertex of the network, with the bin probabilities taken from fits to yeast data. While not optimal, the use of yeast parameters seemed an acceptable first-pass attempt as, for example, edge distributions from metabolic networks (which also follow power-law behavior) have been shown to be very similar across species (Jeong et al., 2000 Nature 407:651-4). Ideally, one would want to acquire distributions for a number of species; however, it appears that the lack of reasonably large data sets could be a hindrance, with improper edge distributions perhaps masking interactions that would otherwise be apparent, particularly in interspecies predictions. Thus, parameters will be used from a well-characterized system (e.g. yeast) in a distribution with identical mean but with greater variance. This requirement can be fulfilled with the incorporation of the negative multinomial distribution (instead of the multinomial distribution) into simulations, defined as

$$P(n_1, n_2, \ldots n_k) = \frac{\Gamma\left(N + \sum_{i=1}^{k} n_i\right)}{\left(\prod_{i=1}^{k} n_i!\right)\Gamma(N)} Q^{-N} \prod_{i=1}^{k}\left(\frac{P_i}{Q}\right)^{n_i}\ ;\ \left(n_j \geq 0\right) \quad (12)$$

However, while one can match the expected value, one can only generate a variance that is greater than, but not equal to, the multinomial's. This is because a negative binomial distribution tends to a Poisson distribution as the variance decreases, and the Poisson distribution has typically larger variance than a multinomial distribution with the same mean.

From an implementation standpoint, this approach, while capable of handling large networks, benefits significantly from the use of appropriate computational resources. Running a C programming language implementation of the method of the invention, which proved to be significantly more rapid than our previous implementations in Matlab. In addition, it is beneficial to have a 5-node Beowulf cluster running Linux, with each node having 2, 1GHz CPOs. The availability of appropriate hardware and software was invaluable, as it can take a considerable amount of time to establish a stationary distribution (1-2 days in this case) and to generate the posterior (many days to generate a posterior with resolution of low-probability edges).

One means for improving the method of the invention is to implement "repulsive" interactions between domains. This can be achieved by assigning domain-domain interaction probabilities of < 0.5 to interactions that are never present. While requiring careful normalization and balancing with "attractive" probabilities, this feature should provide enhanced resolution of predicted interactions (bigger peaks and deeper valleys in the posterior plots). While having its own set of favorable and unfavorable properties, two-hybrid data should prove particularly valuable for this approach.

8. <u>EXAMPLE: AN ADVANCED METHOD FOR ESTIMATING PARAMETERS</u>

The following example exemplifies a preferred embodiment of the invention. In this example, the following equation;

$$\widehat{p}_{ij} = \sum_{r \in v_i} \sum_{s \in v_j} \frac{\widehat{p}(d_r, d_s)}{|v_i||v_j|}, \quad \widehat{p}(d_r, d_s) = \frac{1}{2}(1 + \frac{n_{rs}^+}{n_r n_s + \Psi}), \quad (13)$$

was abandoned, and a more advanced method for estimating parameters $\{p_{ij}\}$ and p $(d_r, d_s)$ that allows extracting at least twice as much information from the training data as the older method was used.

In this example, three different levels of protein structure in computation of *P*(local) are considered. The highest level corresponds to Pfam domains. These domains are specific, relatively long in length, and thus occur quite rarely within a given population of proteins. *Using Saccharomyces cerevisiae* (baker's yeast) proteome as a test system, an attempt was made to characterize a protein with at least one Pfam domain using the HIMMER-2 package (Eddy, 1998 Bioinformatics 14:755-63) at 0.001 e-value cutoff level. For the dataset containing 1771 proteins known to have at least one interaction (Stanley Field's lab homepage; http://depts.washington.edu/sfields), it was possible to provide Pfam domains for approximately 30 per cent of all yeast proteins.

The lowest level corresponds to four adjacent amino acids observed in a running window which spanned the entire length of the protein. To reduce the size of feature space the twenty amino acids were mapped to a smaller alphabet consisting of six "letters" (Table 1). This mapping is based on the amino acid groupings of Taylor (1993, J Theor Biol 164:65-83), with amino acids sharing similar physicochemical properties being grouped together. From this mapping it was possible to generate $6^4$=1296 unique four-tuples.

Table 1. Mapping of amino acids to group numbers. Feature class describes the general properties of the group.

| Amino Acid | Group Number | Feature Class |
|---|---|---|
| I, V, L, M | 1 | Mostly Aliphatic |
| F, Y, W | 2 | Aromatic |
| H., K, R | 3 | Positive Charge |
| D, E | 4 | Negative Charge |
| Q, N, T, P | 5 | Misc. |
| A, G, C, S | 6 | Small Size |

The intermediate-scale feature consisted of ten adjacent amino acids, again taken from a running window spanning the entire protein. In this instance, six particular traits were looked at including positive charge, negative charge, hydrophobicity, amphipathy, proline-richness, and serine-richness. Each ten-mer was analyzed for the density of each of these traits, and a six-element feature vector generated containing the strength of each of the six traits. From this feature vector, the two best-represented traits were chosen to represent this particular ten-mer, with vectors containing multiple identical top traits having the top two features chosen at alphabetical order. Note that this representation is not specific as to the order of amino acids within a 10-mer, but rather first describes the density of each of a set of traits and then assigns only the best two to the ten-mer. The scales and features described here could easily have been chosen in a variety of different biologically relevant ways. For instance, the intermediate scale could be redefined, for example, with specific degrees of hydrophobicity, surface tension, etc. determined for each amino acid as well as for each ten-mer. While the features chosen are not necessarily optimal the current implementation provides sufficient detail for this level of analysis.

Table 2. Determination of score for classes of amino acids found in 10-mer window.
'x' is the number of amino acids that fall into a given feature class.

| Feature Class | Score | | |
|---|---|---|---|
| | 0 | 1 | 2 |
| Pos., Neg, Aliphatic | $x < 3$ | $3 \leq x \leq 6$ | $x \geq 6$ |
| Hydrophobic | $x < 4$ | $4 \leq x < 7$ | $x \geq 7$ |
| proline-rich | $x \leq 1$ | $1 < x < 4$ | $x \geq 4$ |
| Serine-rich | $x \leq 1$ | $1 < x < 3$ | $x \geq 3$ |

Probabilities of interactions were assigned as follows. As described earlier, it was assumed that some set of features is responsible for the observed interaction within each protein in an interacting pair. To determine the specific pairs of features giving rise to the interaction, and then translate this information into a prediction for proteins for which there is no information, training data consisting of a large number of known interactions between proteins with known sequences was used. Using this training data an attempt was made to infer whether an interaction exists between a set of proteins based upon analyzing the feature set of these potentially interacting proteins. Note that the assignment of protein-protein interaction probabilities is related to previous work, but now makes use of both positive (existing interactions) and negative (absence of interactions) information from the training data, while other versions of the approach described herein discarded the negative information.

The model was set so that, in the absence of interaction data, any pair of features will interact with a probability of 0.5. In this model the probability of feature-feature interaction less than 0.5 indicates that the features "repel," while probability greater than 0.5 indicates that the features "attract.". The probability of a pair of features $(d_i, d_j)$ interacting with one another is estimated in the following way

$$\hat{p}(d_i, d_j) = \frac{n_{ij}^+ + \Psi/2}{n_{ij}^+ + \gamma n_{ij}^- + \Psi} \qquad (14)$$

where $n_{ij}^+$ is the number of times feature i is seen in an interaction with feature $j$ (the number of times two interacting proteins have features i and $j$, respectively), and $n_{ij}^-$ is the number of times feature i is not seen in an interaction with feature $j$ (the number of times two non-interacting proteins display features i and $j$, respectively). Finally, and $\gamma$ is a parameter that reflects non-uniform distribution of different types of data, negative and positive, and has direct relation to concept called "boosting" in computer science. To avoid complications with zero probabilities when data are missing, values of $\Psi/2$ any $\Psi$ are added to the numerator and denominator of equation 14" respectively, where $\Psi$ is a small positive value (set to 0.01 in this study) ensuring that feature-feature interaction probabilities are set to 0.5 in the absence of data.

To explain the need to use boosting, one can count observations of the negative (no interaction) and positive (presence of interaction) kind in a typical training data set. The number of known edges within a training set is vastly smaller than the number of non-existent edges: there are $V^2$ *possible* directed edges in a typical molecular network with $V$ vertices, but the number of actually *observed* directed edges, $E$, is usually much smaller than $V^2$. Further, consider a pair of hypothetical features, $x$ and $y$, that are distributed among network vertices with uniform densities $\rho_x$ and $\rho_y$ respectively. It follows that the expected number of *existing* edges between vertices, possessing features $x$ and $y$, is equal to $\rho_x \rho_y E$, while the expected number of pairs of vertices having features x and y, but not an edge in common is equal to $\rho_x \rho_y (V^2 - E)$.

Statistical consistency of the model requires that

$$\mathbb{E}[\hat{p}(d_x, d_y)] = \frac{1}{2}, \qquad (15)$$

if features $x$ and $y$ arc non-informative (E[] is operator of taking expectation). This can be achieved by setting the boosting

parameter, $\gamma$, to an appropriate value less than 1. An estimator for $\gamma$ that guarantees this property can be defined as:

$$\hat{\gamma} = \frac{E}{V^2 - E}.$$  (16)

It is quite easy to modify equation 16 to fit networks with undirected edges.

In addition, the way of computing the probability of an interaction between a pair of proteins ($v_i$, $v_j$) given a set of known feature-feature probabilities has been modified. In previous examples a protein-protein interaction probability, $\hat{P}_{ij}$, was calculated as a simple average of feature-feature interaction probabilities, $\hat{p}(d_k,\, d_l)$. In this work, this equation is modified to take into account the relative frequencies of the relevant features in the training data:

$$\hat{p}_{ij} = \frac{\displaystyle\sum_{d_k \in v_i}\sum_{d_l \in v_j} \hat{p}(d_k, d_l)(f_k f_l)^{-1}}{\displaystyle\sum_{d_x \in v_i}\sum_{d_y \in v_j} (f_x f_y)^{-1}}$$  (17)

where $f_i$ is the frequency of feature $i$. Note that the introduced weights correspond to assumption that the features less frequently observed throughout the training data set are more informative.

Next, the training set was analyzed with all three feature sets and the relative information content of each was compared.

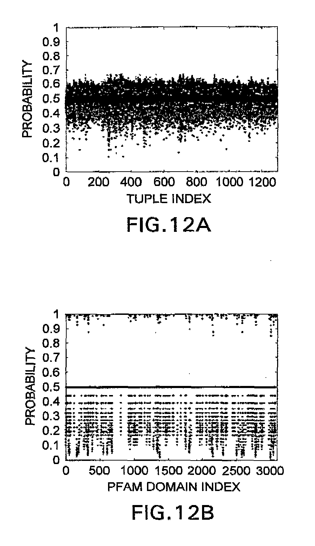

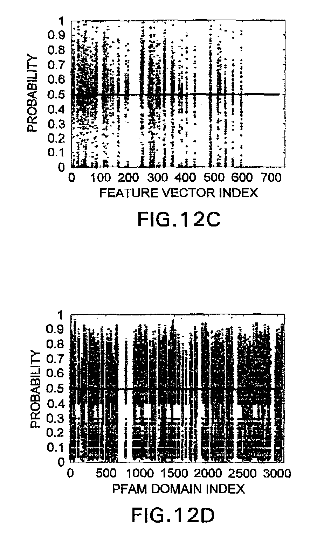

It is expected, that small-scale features such as tuples, will have smaller probabilities of interaction than large-scale features such as Pfam domains. This expectation appears to be true, as shown in Figure 12A. Here, the probability of every tuple-tuple interaction is shown, where for each tuple on the x-axis, the probability of this tuple interacting with each of the other tuples is shown as a scatter plot along the vertical. As is expected in the model, the density of this plot is centered at 0.5. It also appears that tuples provide slightly more negative information (repelling) than positive (attraction) as can be observed by the slight "drifting" of probabilities down towards zero. In contrast, Figure 12B shows probabilities of domain-domain interaction. Note that these interactions are very sparse, in part due to the small number of Pfam domains found when the cutoff E-value was set to 0.001. In total, only 126 protein-protein interactions were found where both proteins had at least 1 domain. At the level of domains, this translated into 448 attractive and 248,553 repulsive domain-domain interactions. As with the plots in this Figure 12, it too is centered at 0.5 since almost all points lie along this line, again indicating that most domain-domain interactions have no supporting information. However, when domains are found in an interaction, knowledge of the types of domains involved provides potentially much stronger probabilities of attraction and repulsion. In fact some pairs show significant strength, residing in the 0.9 - 1 or 0 - 0.1 range. As would be expected by the large number of non-existent edges within the network, negative or repulsive data is predominant. In Figure 12C, features generated by ten-mers are shown. Of the 729 potential features, only 122 are present in the data. However, like Pfam domains, features observed in the data provide a stronger signal of interaction, both positive and negative, than do tuples.

To determine if combining features could help improve an attractive or repulsive signal, the interaction between tuples and Pfam domains was analyzed. As shown in Figure 12D, the combination of features leads to significantly more information than either feature alone. Again, all dots along a vertical line indicate the probability for a given tuple to interact with the domain indicated on the x-axis. While probabilities can only be generated for those proteins having at least one domain, combining features appears to provide much more information than without.

Protein features were defined and characterized at multiple length scales and their probabilities of interaction quantified. To determine the information content of each, so as to better compare them. The probability of protein $v_i$, interacting with protein $v_j$, $\hat{p}\,(v_i,\, v_j)$ is calculated for each of the feature sets described. The total entropy of an observed set of features giving rise to a set of interaction probabilities is determined by applying the following equation:

$$\langle H(p_{ij})\rangle = -\sum_{i,j} p_{ij}\log(p_{ij}),$$  (18)

where $p_{ij}$ is the probability of protein i interacting with protein $j$. Since the uninformative prior probability of any particular interaction is 0.5, the total information content of a set of protein interactions is defined as

$$I = \langle H(0.5) \rangle - \langle H(p_{ij}) \rangle . \qquad (19)$$

Information content for a set of protein interactions based on a given type of feature set is shown in Table 3.

Table 3. Information content for a set of protein interactions generated from given feature interaction probabilities.

| Feature Used | $I$ (bits) |
| --- | --- |
| Tuples | 8,352 |
| Feature Vectors | 17,772 |
| Pfam Domains | 1,263 |
| Old Model | 22 |
| Pfam - Tuple | 6, 700 |
| Theoretical Maximum | 1,569,106 |

Information from all levels can be combined using the following equation:

$$\widehat{p}_{ij} = \frac{\sum_x I_x \widehat{p}_x (v_i, v_j)}{\sum_y I_y} . \qquad (20)$$

A major benefit of the multi-scale protein characterization described here is the ability to use all possible interaction data for training. Previously, characterization of proteins with Pfam domains alone meant that the majority of protein interactions could not be defined; a drawback that has affected other models. While using less stringent E-value cutoffs could perhaps help to increase the number of domains in the network without producing excessive noise, even relatively relaxed cutoffs provide only 60-70% coverage at best.

While the amino acid/feature mappings used for both the four-mer tuples and the ten-mer feature-vectors provided appreciable information, any number of other mappings, subsequence lengths, etc., could have been used. Surprisingly, it was possible to generate significant information with a somewhat arbitrarily chosen feature vector. Undoubtedly, other choices could potentially provide much better performance. The particular mapping and four-mer window used in the generation of tuples was chosen both for its manageable size (mapping the standard alphabet of 20 amino acids directly would generate $20^4$ or 160,000 distinct domains, with potentially $160,000^2$ tuple-tuple interactions to keep track of) as well as for its natural grouping together of amino acids with similar properties. The vector of features (stretches of positive charge, negative charge, hydrophobic, aliphatic, proline, and serine residues) was chosen primarily for proof of concept, with features thought to be of general utility and with some predictive capability. Windows greater than 10 amino acids could obviously be used, and could perhaps be useful in detecting large-scale structures important in establishing interactions between proteins. A related aspect of this approach is the hope that the characterization of features at multiple scales could potentially provide the ability to observe small-scale effects. For instance, it may be possible to observe substitution events that cause a loss or gain of function - generating a novel interaction or deleting an existing one. Characterization with small-scale features would be required for this type of analysis. The determination of which particular types features in the vector were most informative is currently being evaluated.

The present invention is not to be limited in scope by the specific embodiments described herein. Indeed, various modifications of the invention in addition to those described herein will become apparent to those skilled in the art from the foregoing description and accompanying Figures. Such modifications are intended to fall within the scope of the appended claims. Various references are cited herein, the disclosure of which are incorporated by reference in their entireties.

**Claims**

1. A method for identifying the probability of a molecular interaction within a biological network comprising;

   (i) representing molecules as sets of conserved features;
   (ii) computing the attraction probabilities between said features; and
   (iv) using the computation of step (ii) to identify the
   probability of a molecular interaction within a biological network.

2. A method for identifying the probability of protein interactions within a biological network comprising;

   (i) representing proteins as sets of conserved features;
   (ii) computing the attraction probabilities between said features; and
   (iv) using the computation of step (ii) to identify the
   probability of a molecular interaction within a biological network.

3. A method for identifying the probability of protein interactions within a biological network comprising;

   (i) representing proteins as sets of conserved features;
   (ii) computing the attraction probabilities between said features;
   (iii) computing the attraction probabilities between proteins; and
   (iv) using the computation of step (ii) and step (iii) to identify the
   probability of a molecular interaction within a biological network.

4. The method of claim 1, 2 or 3 further comprising computing the topology of a biological network wherein those networks displaying a more biologically realistic topology are assigned a greater probability.

5. The method of claim 1, 2 or 3 wherein the conserved features are protein domains or motifs.

6. The method of claim 1, 2 or 3 wherein the conserved features are nucleic acid molecule motifs.

7. The method of claim 2 or 3 wherein the attraction probabilities between features is computed using equation 6, 14, or 16.

8. The method of claim 2 wherein the attraction probabilities between proteins is accomplished using equation 5 or 17.

9. The method of claim 1 wherein the identification of a probability of a molecular interaction within a biological network is accomplished using equation 1.

10. The method of claim 2 or 3 wherein the identification of a probability of a protein interaction within a biological network is accomplished using equation 1.

11. The method of claim 4 wherein the topology of a biological network is determined using equation 2, 3, 4 or 7.

12. The method of claim 1 further comprising computing the posterior probabilities of interaction between a set of molecules.

13. The method of claim 2 or further comprising computing the posterior probabilities of interaction between a set of proteins.

14. The method of claim 12 or 13 wherein a Markov Chain Monte Carlo technique is used to compute the posterior probabilities of interaction between a set of proteins.

15. The method of claim 12 or 13 wherein equation 1 is used to compute the posterior probabilities of interaction between a set of proteins.

16. A screening method for identification of a compound capable of modifying the interaction between at least two proteins comprising:

(i) identifying an interaction between at least two proteins using the method of claim 2 or 3 ;
(ii) contacting said proteins identified in step (i) with a test compound;
(iii) comparing the interaction of the proteins in the presence of the test compound with the interaction in the absence of the test compound;

wherein a difference in the interaction of the proteins in the presence of the test compound as compared to the interaction in the absence of a test compound indicates identification of a compound capable of modifying the interaction between proteins.

17. A screening method for identification of a compound capable of modifying the interaction between at least two molecules comprising:

(i) identifying an interaction between at least molecules using the method of claim 1 ;
(ii) contacting said molecules identified in step (i) with a test compound;
(iii) comparing the interaction of the molecules in the presence of the test compound with the interaction in the absence of the test compound;

wherein a difference in the interaction of the molecules in the presence of the test compound as compared to the interaction in the absence of a test compound indicates identification of a compound capable of modifying the interaction between molecules.

FIG.1

FIG.2A

FIG.2B

DISTRIBUTION OF CONNECTIVITY FOR
YEAST PROTEIN NETWORK (DIP)

FIG. 3

FIG.4A

FIG.4B

FIG.4C

FIG. 5A

FIG. 5B

FIG. 5C

FIG. 5D

FIG. 6

FIG.7A

FIG.7B

FIG.8

FIG. 9

FIG.10A

FIG.10B

FIG. 11A

FIG. 11B

FIG. 11C

FIG.12A

FIG.12B

FIG.12C

FIG.12D

**PARTIAL EUROPEAN SEARCH REPORT**

which under Rule 63 of the European Patent Convention
shall be considered, for the purposes of subsequent
proceedings, as the European search report

Application Number

EP 08 02 0358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/66067 A (ROSETTA INPHARMATICS INC [US]) 23 December 1999 (1999-12-23)<br>* abstract *<br>* claim 1 *<br>----- | 1-17 | INV.<br>G06F19/00 |
| X | MARCOTTE E M ET AL: "DETECTING PROTEIN FUNCTION AND PROTEIN-PROTEIN INTERACTIONS FROM GENOME SEQUENCES"<br>SCIENCE, AMERICAN ASSOCIATION FOR THE ADVANCEMENT OF SCIENCE,, US,<br>vol. 285, 30 July 1999 (1999-07-30), pages 751-753, XP000891681<br>ISSN: 0036-8075<br>* abstract *<br>----- | 1-17 | |

TECHNICAL FIELDS
SEARCHED (IPC)

G06F

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 11 March 2009 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches
Patentamt

European
Patent Office

Office européen
des brevets

**INCOMPLETE SEARCH
SHEET C**

Application Number

EP 08 02 0358

Claim(s) searched incompletely:
      1-17

Reason for the limitation of the search:

Claims 1 to 3 have been drafted as separate independent method claims. Under Article 84 in combination with Rule 43(2) EPC an application may contain more than one independent claim in a particular category only if the subject-matter claimed falls within one or more of the exceptional situations set out in paragraphs (a), (b) or (c) of Rule 43(2) EPC. This is not the case in the present application because the subject-matter of the application does neither involve a plurality of inter-related products, nor different uses of a product or apparatus nor alternative solutions to a particular problem. In contrast, the above mentioned claims appear to relate effectively to very similar subject-matter and to differ from each other only with regard to the definition of the subject-matter for which protection is sought. Therefore, lack of clarity of the claims as a whole arises, since the plurality of independent claims makes it difficult, if not impossible, to determine the matter for which protection is sought, and places an undue burden on others seeking to establish the extent of the protection (Article 84 EPC).

Furthermore, independent method claims 1 to 3 are each unclear (Article 84 EPC) because these claims attempt to define the subject-matter in terms of the results to be achieved, which merely amounts to statements of the underlying problems, without providing the technical features necessary for achieving these results. The expressions "representing molecules/proteins as sets of conserved features", "computing the attraction probabilities between said features/proteins" and "identify the probablity of a molecular interaction within a biological network" are examples for such statements of the underlying problems.

Moreover, some terms used in the independent method claims 1 to 3 have no well-recognised meaning and leave the reader in doubt as to the meaning of the technical features to which they refer, thereby rendering the definition of the subject-matter of said claims unclear (Article 84 EPC). For example, terms like "conserved features" and "attraction probabilities" have no well-recognised technical meaning.

Independent method claim 1 is not supported by the description as required by Article 84 EPC as its scope is broader than justified by the description and drawings. The described embodiments are directed to protein interaction networks and to protein domains or motifs as conserved features of given proteins. However, the wording of claim 1 which relates to molecules, is too broad and vague and does not enable the skilled person in the art to carry out the invention without any further inventive effort.

It is clear from the description and from figure 1 that the the present invention discloses a computer-implemented statistical model, based on the knowledge of known protein interaction networks, which assigns probabilities to all possible networks formed from a fixed number of vertices. However, independent method claims 1 to 3 try to identify the probability of a molecular/protein interaction within a biological

Europäisches
Patentamt

European
Patent Office

Office européen
des brevets

INCOMPLETE SEARCH
SHEET C

Application Number

EP 08 02 0358

network. This striking inconsistency between the independent claims and the description leads to doubts concerning the matter for which protection is sought, thereby rendering the claims unclear (Article 84 EPC).

The screening method claims 16 and 17 are unclear because they define the step of "identifying an interaction between at least two molecules/proteins using the method of claim 1, 2 or 3". However, independent method claims 1 to 3 only identify the probability of a molecular interaction within a biological network and do not identify an interaction between at least two molecules/proteins, thereby rendering the definition of the subject-matter of said claims unclear (Article 84 EPC).

Therefore, the Search Division cannot clearly determine which technical problem with which technical features is to be  solved by the present set of claims. This non-compliance with the substantive provisions of the Convention is to such an extent that only a partial search report is drawn up (Rule 63 EPC and the Guidelines, B-VIII, 3) taking into account of the following: The only technical teaching which the skilled person is able to derive from the whole application is the computer-implemented prediction of a most likely protein interaction network for a given set of  proteins.

However, such a general technical teaching is well known to the skilled person as can be seen from the documents of the search report.

**ANNEX TO THE EUROPEAN SEARCH REPORT
ON EUROPEAN PATENT APPLICATION NO.**

EP 08 02 0358

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2009

| Patent document cited in search report | | Publication date | Patent family member(s) | | Publication date |
|---|---|---|---|---|---|
| WO 9966067 | A | 23-12-1999 | AU | 750741 B2 | 25-07-2002 |
| | | | AU | 4693599 A | 05-01-2000 |
| | | | CA | 2335187 A1 | 23-12-1999 |
| | | | EP | 1086240 A1 | 28-03-2001 |
| | | | JP | 2002518021 T | 25-06-2002 |
| | | | US | 6132969 A | 17-10-2000 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **UETZ et al.** *Nature,* 2000, vol. 403, 623-627
- **XENARIOS et al.** *Nucleic Acid Res.,* 2000, vol. 28, 289-291
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **KROGH et al.** *J. Mol. Biol.,* 1994, vol. 235, 1501-1531
- **GRUNDY et al.** *Biochem Biophys. Res. Commun,* 1997, vol. 231, 760-6
- **WU et al.** *Comput. Appl. Biosci,* 1996, vol. 12, 109-118
- **NEUWALD et al.** *Protein Sci.,* 1995, vol. 4, 1618-1632
- **NEUWALD.** *Nucleic Acids Res.,* 1997, vol. 25, 1665-1677
- **KAZIC.** Molecular Modeling: From Virtual Tools to Real Problems. American Chemical Society, 1994, 486-494
- New Data Challenges in Our Information Age Glaesar. **KAZIC.** Proceedings of the Thirteenth International CODATA Secretariat. 1994, C133-C140
- **GOTO et al.** *Pac. Symp. Biocomput.,* 1997, 175-186
- **BONO et al.** *Genome Res.,* 1998, vol. 8, 203-210
- **SELKOV et al.** *Nucleic Acids Res.,* 1996, vol. 24, 26-28
- **KOIKE ; RZHETSKY.** *Gene,* 2000, vol. 259, 235-244
- **BATEMAN et al.** *Nucleic Acids Res.,* 2000, vol. 28, 263-6
- **ALBERT et al.** *Nature,* 2000, vol. 406, 378-382
- **BARABASI ; ALBERT.** *Science,* 1999, vol. 286, 509-512
- **XENARIOS et al.** *Nucleic Acids Research,* 2000, vol. 28, 289-291

- **BATEMAN et al.** *Nucleic Acids Research,* 2000, vol. 28, 263-6
- Markov chain Monte Carlo in Practice. Chapman & Hall
- **HASTINGS.** *Biometrika,* 1970, vol. 57, 97-109
- **GREEN.** *Biometrika,* 1995, vol. 57 (82), 711-732
- **GOTO et al.** *Pac Symp Biocomput,* 1997, 175-86
- **BENEDICT et al.** *J. Biochem Chem,* 2000, vol. 275, 8461-8
- **HU et al.** *J. Biol Chem,* 1998, vol. 273, 33489-94
- **VON DASSOW et al.** *Nature,* 2000, vol. 406, 188-92
- **JEONG et al.** *Nature,* vol. 411, 41-42
- **JEONG et al.** *Nature,* 2000, vol. 407, 651-4
- **XENARIOS et al.** *Nucleic Acids Research,* 2001, vol. 28, 289-91
- **ITO et al.** *Proc. Natl. Acad Sci. USA,* 2000, vol. 97, 1143-7
- **UETZ et al.** *Nature,* 2000, vol. 403, 623-7
- **HUTT et al.** *J. Biol Chem,* 2000, vol. 275, 18511-9
- **WU et al.** *J. Biol. Chem,* 1998, vol. 273, 26931-26938
- **ULLRICH et al.** *J. Biol. Chem,* 1993, vol. 268, 18143-50
- **BALLINGER et al.** *Mol Cell Biol,* 1999, vol. 19, 4535-45
- **AWATA et al.** *J. Biol. Chem,* 2001, vol. 4, 4
- **GILKS et al.** Markov chain Monte Carlo in Practice. Chapman & Hall/CRC, 1996
- **GREEN.** *Biometrika,* 1995, vol. 82, 711-732
- **EDDY.** *Bioinformatics,* 1998, vol. 14, 755-63
- **TAYLOR.** *J Theor Biol,* 1993, vol. 164, 65-83